# EUROPEAN PATENT SPECIFICATION

(11) **EP 2 170 793 B1**
(45) Date of publication and mention of the grant of the patent: **10.08.2016**
(21) Application number: 08774176.5
(22) Date of filing: 20.06.2008
(51) Int. Cl.: C07C 29/62, C07C 31/20, C07C 31/22, C07C 31/36, C07D 303/08, C08G 59/02, C07D 301/26

(54) **MANUFACTURE OF EPICHLOROHYDRIN**
HERSTELLUNG VON EPICHLOROHYDRIN
FABRICATION D'ÉPICHLOROHYDRINE

(30) Priority: 28.06.2007 FR 0756125; 14.12.2007 US 13710
(43) Date of publication of application: 07.04.2010
(73) Proprietor: SOLVAY SA, 1120 Bruxelles (BE)
(72) Inventor: KRAFFT, Philippe, B-1640 Rhode Saint Genese (BE); GILBEAU, Patrick, B-7090 Braine-le-comte (BE)
(74) Representative: Vande Gucht, Anne
(86) International application number: PCT/EP2008/057876
(87) International publication number: WO 2009/000773

(56) References cited:
- WO-A-2005/021476
- WO-A-2005/054167
- WO-A-2006/020234
- WO-A-2006/100314
- US-A1- 2001 014 763

## Description

The present invention relates to a process for manufacturing epichlorohydrin from dichloropropanol.

Dichloropropanol is a reaction intermediate in the manufacture of epichlorohydrin and epoxy resins (Kirk-Othmer Encyclopedia of Chemical Technology, Fourth Edition, 1992, Vol. 2, page 156, John Wiley & Sons, Inc.).

According to known processes, dichloropropanol can be obtained in particular by hypochlorination of allyl chloride, by chlorination of allyl alcohol and by hydrochlorination of glycerol.

The latter process exhibits the advantage that the dichloropropanol can be obtained starting from renewable raw materials. It is known that petrochemical natural resources, from which the fossil materials originate, for example oil, natural gas or coal, available on Earth are limited.

The process of hydrochlorination of glycerol has a selectivity which leaves much to be desired, which results in the formation of by-products that are difficult to eliminate. This requests eventually also an extensive purification of the starting raw materials.

The invention aims to solve this problem by providing a new process for manufacturing epichlorohydrin.

The invention therefore relates to a process for manufacturing epichlorohydrin, by subjecting dichloropropanol to a dehydrochlorination reaction in which, at least one part of the dichloropropanol was obtained by reacting a glycerol-based product with a chlorinating agent, wherein the glycerol-based product contains at least 900 g/kg of glycerol and from 0.001 g/kg to 5 g/kg of 1,3-propanediol.

One of the essential characteristics of the present invention resides in the identification of glycerol impurities which although they interfere with the operations for separation and treatment of the effluents from the processes employing glycerol, the said glycerol can nevertheless be used as raw material in the manufacture of epichlorohydrin.

In the process -according to the invention, the glycerol-based product may also contain, in addition, 1,2-ethanediol (ethylene glycol), 1,2-propanediol (propylene glycol), or a mixture of these two compounds. These compounds may come from the glycerol manufacturing processes such as the fermentation of mono- and polysaccharides derived from biomass or the hydrogenation of monosaccharides and derived alcohols.

In the process according to the invention, the glycerol content of the glycerol-based product is greater than or equal to 900 g/kg, in most cases greater than or equal to 950 g/kg, often greater than or equal to 990 g/kg, frequently greater than or equal to 995 g/kg, particularly greater than or equal to 999 g/kg and specifically greater than or equal to 999.5 g/kg. A glycerol content greater than or equal to 999.9 g/kg is convenient.

In the process according to the invention, the 1,3-propanediol content of the glycerol-based product is less than or equal to 5 g/kg, frequently less than or equal to 1 g/kg, particularly less than 0.7 g/kg, specifically less than or equal to 0.5 g/kg and often less than or equal to 0.2 g/kg. This mount is greater than or equal to 0.001 g/kg, generally greater than or equal to 0.005 g/kg, commonly greater than or equal to 0.01 g/kg, in many cases greater than or equal to 0.04 g/kg and particularly greater than or equal to 0.1 g/kg.

In the process according to the invention, the ethylene glycol content of the glycerol-based product is generally less than or equal to 100 g/kg of product. This content is usually less than or equal to 90 g/kg, commonly less than or equal to 50 g/kg, in many cases less than or equal to 10 g/kg, particularly less than or equal to 1 g/kg, specifically less than or equal to 0.5 g/kg and often less than or equal to 0.2 g/kg. This amount is usually greater than or equal to 0.001 g/kg of product, generally greater than or equal to 0.005 g/kg, commonly greater than or equal to 0.01 g/kg, in many cases greater than or equal to 0.04 g/kg and often greater than or equal to 0.1 g/kg.

In the process according to the invention, the propylene glycol content of the glycerol-based product is generally less than or equal to 100 g/kg of product. This content is usually less than or equal to 90 g/kg, commonly less than or equal to 50 g/kg, in many cases less than or equal to 10 g/kg, particularly less than or equal to 1 g/kg, specifically less than or equal to 0.5 g/kg and often less than or equal to 0.2 g/kg. This amount is usually greater than or equal to 0.001 g/kg of product, generally greater than or equal to 0.005 g/kg, in many cases greater than or equal to 0.01 g/kg, commonly greater than or equal to 0.04 g/kg and often greater than or equal to 0.1 g/kg.

In the process according to the invention, the glycerol-based product generally contains glycerol alkyl ethers such as described in Application WO2007/144335 in the name of Solvay SA, more specifically from page 2, line 6 to page 3, line 25.

The content of glycerol alkyl ethers of the glycerol-based product is generally less than or equal to 90 g/kg of product, usually less than or equal to 50 g/kg, in many cases less than or equal to 10 g/kg, commonly less than or equal to 5 g/kg, particularly less than or equal to 1 g/kg, specifically less than or equal to 0.5 g/kg and often less than or equal to 0.2 g/kg. This amount is usually greater than or equal to 0.001 g/kg, generally greater than or equal to 0.005 g/kg, commonly greater than or equal to 0.01 g/kg, in many cases greater than or equal to 0.04 g/kg and often greater than or equal to 0.1 g/kg.

The glycerol alkyl ethers are generally selected from methyl, ethyl, propyl, butyl ethers and any mixtures of at least two of them, usually from methyl ethers, and in many cases from monomethyl ethers. Such ethers are for examples 2-methoxy-1,3-propanediol, 3-methoxy-1,2-propanediol, and mixtures thereof.

In the process according to the invention, the glycerol-based product may also comprise monoalcohols, such as the monoalcohols described in Application WO2007/144335 in the name of Solvay SA, more specifically from page 3, lines 26 to 31.

These monoalcohols can be present in an amount generally greater than or equal to 0.001 g/kg of product, usually greater than or equal to 0.01 g/kg, commonly greater than or equal to 0.1 g/kg, in many cases greater than or equal to 0.5 g/kg, and often greater than or equal to 1 g/kg. This amount is generally less than or equal to 20 g/kg of product, usually less than or equal to 10 g/kg, commonly less than or equal to 5 g/kg and in many cases less than or equal to 2 g/kg.

These monoalcohols are usually selected from methanol, ethanol, n-propanol, isopropanol, butanol, and any mixture of at least two of them, generally from methanol, ethanol, and mixtures thereof, and the monoalcohol is often methanol.

In the process according to the invention, the glycerol-based product may also comprise water in an amount generally greater than or equal to 0.01 g/kg of product and less than or equal to 100 g/kg. This amount is usually less than or equal to 50 g/kg, commonly less than or equal to 40 g/kg, in many cases less than or equal to 30 g/kg, particularly less than or equal to 20 g/kg, specifically less than or equal to 10 g/kg, often less than or equal to 5 g/kg and frequently less than or equal to 2 g/kg. This amount is usually higher than or equal to 0.05 g/kg, commonly higher than or equal to 0.1 g/kg, in many cases higher than or equal to 0.5 g/kg, and particularly higher than or equal to 1 g/kg.

In the process according to the invention, the glycerol-based product according to the invention may also comprise ketones, aldehydes, alkyl esters of fatty acids, glycerol esters, carboxylic acids, glycerol oligomers and salts such as described in Application WO2007/144335 in the name of Solvay SA, more specifically, from page 5, lines 12 to 20.

The ketone can be selected from acetone, hydroxyacetone and dihydroxyacetone.

These ketones are generally present in an amount greater than or equal to 0.001 g/kg of product and usually greater than or equal to 0.005 g/kg. This amount is generally less than 10 g/kg of product, usually less than or equal to 5 g/kg of product, commonly less than or equal to 1 g/kg of product, in many cases less than or equal to 0.5 g/kg of product, particularly less than or equal to 0.1 g/kg of product and specifically less than or equal to 0.01 g/kg of product.

The aldehyde can be selected from acetaldehyde, propionaldehyde, butyraldehyde, acrolein, glyceraldehyde and any mixture of at least two of them.

These aldehydes are generally present in an amount greater than or equal to 0.001 g/kg of product and usually greater than or equal to 0.005 g/kg. This amount is generally less than 10 g/kg of product, usually less than or equal to 5 g/kg of product, commonly less than or equal to 1 g/kg of product, in many cases less than or equal to 0.5 g/kg of product, particularly less than or equal to 0.1 g/kg of product and specifically less than or equal to 0.01 g/kg of product.

The esters can be selected from alkyl esters of fatty acids, glycerol esters, such as, for example, mono- and diglycerides, and any mixture thereof. Alkyl esters of fatty acids may be selected from alkyl palmitate, alkyl oleate, alkyl linoleate, alkyl stearate, and any mixture of at least two of them. Methyl esters are more often encountered. Glycerol esters may be selected from glycerol acetate, glycerol mono palmitate, mono oleate, mono linoleate, mono linolenate, mono stearate and any mixture of at least two of them.

These esters are generally present in an amount greater than or equal to 0.001 g/kg of product, usually greater than or equal to 0.1 g/kg, commonly greater than or equal to 0.5 g/kg, in many cases greater than or equal to 1 g/kg and often greater than or equal to 2 g/kg. This amount is generally less than 50 g/kg of product, usually less than or equal to 30 g/kg of product, commonly less than or equal to 20 g/kg of product, in many cases less than or equal to 15 g/kg of product, particularly less than or equal to 10 g/kg of product and specifically less than or equal to 5 g/kg of product.

The carboxylic acids are usually fatty acids. They can be selected from palmitic, oleic, linoleic, linolenic, stearic acid, and any mixture of at least two of them.

These acids are generally present in an amount greater than or equal to 0.001 g/kg of product, usually greater than or equal to 0.1 g/kg, commonly greater than or equal to 0.5 g/kg, in many cases greater than or equal to 1 g/kg and often greater than or equal to 2 g/kg. This amount is generally less than 50 g/kg of product, usually less than or equal to 30 g/kg of product, commonly less than or equal to 20 g/kg of product, in many cases less than or equal to 15 g/kg of product, particularly less than or equal to 10 g/kg of product and specifically less than or equal to 5 g/kg of product.

In the process according to the invention, the glycerol-based product according to the invention may also comprise glycerol oligomers, such as described in Patent Application FR 08/52206 in the name of Solvay SA, more specifically from page 2, line 6 to page 3, line 20.

By glycerol oligomers, one intends to denote compounds resulting from condensation reactions between at least two glycerol molecules.

In the process according to the invention the glycerol oligomer which can be present in the glycerol-based product results generally from condensation reactions between at least two glycerol molecules (dimer of glycerol) and at most seven glycerol molecules (heptamer of glycerol), usually at most six glycerol molecules (hexamer of glycerol), commonly at most five glycerol molecules (pentamer of glycerol), in many cases at most four glycerol molecules (tetramer of glycerol), and often at most three glycerol molecules (trimer of glycerol).

The glycerol oligomer is frequently a compound resulting from the condensation of two glycerol molecules.

The glycerol oligomer can be selected from oligomers exhibiting a linear structure, a branched structure, a cyclic structure, and any mixture of at least two of them.

By oligomers exhibiting a linear structure, one intends to denote oligomers in the molecule of which all the carbon atoms are located in only one atoms chain, which does not constitute a cycle.

By oligomers exhibiting a branched structure, one intends to denote oligomers in the molecule of which the carbon atoms are located in at least two atoms chains.

By oligomers exhibiting a cyclic structure, one intends to denote oligomers in the molecule of which at least one part of the carbon atoms are located in at least one cycle. The number of carbon atoms constituting the cycle is generally higher than or equal to 6, usually higher than or equal to 7, and commonly higher than or equal to 8. The number of carbon atoms constituting the cycle is generally lower than or equal to 20, usually lower than or equal to 15, and commonly lower than or equal to 10. The cycle comprises generally at least 2 oxygen atoms. The oligomers of glycerol with a cyclic structure, comprising only one cycle containing 6, 7 or 8 atoms, of which 2 atoms are oxygen atoms are often encountered.

The glycerol oligomer is often chosen from dimers of glycerol, trimers of glycerol, tetramers of glycerol, and any mixture of at least two of them. The glycerol oligomer is often a dimer of glycerol.

The dimer of glycerol is generally a mixture of dimers of glycerol, comprising one dimer with a linear structure, at least one dimer with a branched structure and at least one dimer with a cyclic structure. The dimer with the cyclic structure comprises generally at least one cycle and usually only one cycle. That cycle comprises generally 6 atoms, usually 7 atoms and commonly 8 atoms, of which 2 atoms are oxygen atoms, the others atoms being carbon atoms.

The glycerol oligomer is generally a mixture of 3-(2,3-dihydroxy-propoxy)-propane-1,2-diol, 3-(2-hydroxy-1-hydroxymethyl-ethoxy)-propane-1,2-diol, 2-(2-hydroxy-1-hydroxymethyl-ethoxy)-propane-1,3-diol), of cis- and trans-2,5-bis-hydroxymethyl-1,4-dioxane, of cis- et trans-2,6-bis-hydroxymethyl-1,4-dioxane, de cis- et trans-6-hydroxy-2-hydroxymethyl-1,4-dioxepane, and of cis- et trans-3,7-dihydroxy-1,5-dioxocane.

The glycerol oligomer is usually a glycerol oligomer with a cyclic structure.

The glycerol oligomer can be selected from dimers of glycerol with a linear structure, dimers of glycerol with a branched structure, and any mixture thereof.

In the following description, glycerol oligomers will also be named polyglycerols and dimers, trimers and tetramers of glycerol will also be named diglycerols, triglycerols and tetraglycerols.

In the glycerol based product used in the process according to the invention, the polyglycerol content is generally lower than or equal to 10 g/kg of glycerol-based product, usually lower than or equal to 5 g/kg, commonly lower than or equal to 2.5 g/kg, in many cases lower than or equal to 1 g/kg, particularly lower than or equal to 0.1 g/kg. This content is usually higher than or equal to 0.05 g/kg.

In the glycerol based product used in the process according to the invention, the diglycerol content is generally lower than or equal to 10 g/kg of glycerol-based product, usually lower than or equal to 5 g/kg, commonly lower than or equal to 2.5 g/kg, in many cases lower than or equal to 1 g/kg, particularly lower than or equal to 0.1 g/kg. This content is usually higher than or equal to 0.05 g/kg.

In the glycerol based product used in the process according to the invention, the content of 3-(2,3-dihydroxy-propaxy)-propane-1,2-diol is generally lower than or equal to 10 g/kg of glycerol-based product, usually lower than or equal to 5 g/kg, commonly lower than or equal to 2.5 g/kg, in many cases lower than or equal to 1 g/kg, particularly lower than or equal to 0.1 g/kg. This content is usually higher than or equal to 0.05 g/kg.

In the glycerol based product used in the process according to the invention, the sum of the contents of 3-(2-hydroxy-1-hydroxymethyl-ethoxy)-propane-1,2-diol and 2-(2-hydroxy-1-hydroxymethyl-ethoxy)-propane-1,3-diol is generally lower than or equal to 10 g/kg of glycerol-based product, usually lower than or equal to 5 g/kg, commonly lower than or equal to 2.5 g/kg, in many cases lower than or equal to 1 g/kg, particularly lower than or equal to 0.1 g/kg. This content is usually higher than or equal to 0.05 g/kg.

In the glycerol based product used in the process according to the invention, the sum of the contents of cis- et trans-2,5-bis-hydroxymethyl-1,4-dioxane, cis- et trans-2,6-bis-hydroxymethyl-1,4-dioxane, cis- et trans-6-hydroxy-2-hydroxymethyl-1,4-dioxepane, et cis- et trans-3,7-dihydroxy-1,5-dioxocane is generally lower than or equal to 10 g/kg of glycerol-based product, usually lower than or equal to 5 g/kg, commonly lower than or equal to 2.5 g/kg, in many cases lower than or equal to 1 g/kg, particularly lower than or equal to 0.1 g/kg. This content is usually higher than or equal to 0.05 g/kg.

In the glycerol based product used in the process according to the invention, the salts can be chosen from alkaline metal chlorides, alkaline metal sulfates, alkaline metal phosphates, alkaline-earth metal chlorides, alkaline-earth metal sulfates, alkaline-earth metal phosphates, and any mixtures of at least two of them.

The alkaline metal is generally sodium, usually potassium and commonly a mixture thereof. The alkaline-earth metal is generally calcium, usually magnesium and commonly a mixture thereof. Mixtures of sodium and potassium chlorides and/or sodium and potassium sulfates are generally encountered.

The salt can be present in an amount generally greater than or equal to 0.0005 g/kg of product, usually greater than or equal to 0.001 g/kg and commonly greater than or equal to 0.01 g/kg. This amount is generally less than or equal to 10 g/kg of product, usually less than or equal to 1 g/kg of product, and commonly less than or equal to 0.1 g/kg of product.

The glycerol-based product used in the process for manufacturing epichlorohydrin according to the invention may contain a nitrogen compound.

In the process according to the invention, the glycerol-based product according to the invention may also comprise contain a nitrogen compound, such as described in Patent Application FR 07/59891 in the name of Solvay SA, more specifically from page 2, line 17 to page 3, line 20.

Molecular nitrogen is not considered as a nitrogen compound.

The nitrogen compound can be an inorganic or an organic compound. By inorganic compound, one intends to designate compound the molecule of which does include neither a carbon-carbon bond, nor a carbon-hydrogen bond. By organic compound, one intends to designate a compound the molecule of which includes at least one carbon-carbon bond or one carbon-hydrogen bond.

The nitrogen inorganic compound can be selected from ammonia, hydrazine, chloramines, ammonium inorganic salts, nitrates, nitrites, cyanates, isocyanates, isothiocyantes of metals or ammonium, and any mixture of at least two of them.

The nitrogen organic compound can be selected from nitrogen compounds present in cells of plant origin, generally among amines, urea, proteins, peptides, aminoacids,glucosinolates and their degradation products (isothiocyantes, thiocyanates, nitriles, oxazolidinethiones), phospholipids containing nitrogen, chlorophyll, sinapine, and any mixtures of at least two of them.

Examples of phospholipids containing nitrogen are phospatidyl chloline, phosphatidyl serine and phosphatidyl ethanolamine.

Aminoacids free or able to be included in peptides or proteins cane be selected from alanine, arginin, aspartic acid, cystein, glutamic acid, glycin, histidin, isoleucin, leucin, lysin, methionin, phenylalanin, prolin, serin, threonin, trytptophane, tyrosin, valin, and any mixture of at least two of them.

Glucosinolates can be selected from sinigri, gluconapin, glucobrassicanapin, glycorucin, glucoberteroin, glucoraphanin, glucoalyssin, gluconasturtiin, progoitrin, napoleiferin, glucobrassicin, neoglucobrassicin, and any mixture of at least two of them.

In the glycerol based product used in the process according to the invention, the total content of nitrogen compound expressed as elemental nitrogen is generally lower than or equal to 1 g of N/kg, usually lower than or equal to 0.5 g of N/kg, commonly lower than or equal to 0.1 g of N/kg, in many cases lower than or equal to 0.05 g of N/kg, particularly lower than or equal to 0.03 g of N/kg and specifically lower than or equal to 0.01 g of N/kg. This content is usually higher than or equal to 0.1 mg N/kg.

The glycerol based product used in the process according to the invention can also contain metallic and non-metallic elements chosen from Al, As, Be, Ca, Cd, Co, Cr, Cu, Fe, Hg, Na, Ni, P, Pb, S, Sb, Se, Sn, Te, Ti, V, Zn, and any mixture thereof. The total content of those elements is usually lower than or equal to 0.05 g/kg, generally lower than or equal to 0.035 g/kg, commonly lower than or equal to 0.025 g/kg, in many cases lower than or equal to 0.015 g/kg and particularly lower than or equal to 0.005 g/kg. That total content is usually higher than or equal to 0.1 mg/kg.

The glycerol alkyl ethers, monoalcohols, water, alkyl esters of fatty acids, fatty acids, glycerol esters, glycerol oligomers and salts may be by-products of glycerol manufacturing processes such as, for example, the processes for conversion of oils or fats of vegetable or animal origin via transesterification, saponification or hydrolysis reactions.

The glycerol-based product used in the process for manufacturing epichlorohydrin according to the invention may be manufactured by conversion of saccharides and polysaccharides derived from biomass such as, for example, fermentation and/or hydrogenation processes, carried out under conditions such that the 1,3-propanediol was formed and was not separated from the glycerol. Biomass comprises harvest products, trees, grass, harvest residues, forest residues, animal waste and solid municipal waste.

Alternatively, the glycerol-based product used in the process for manufacturing epichlorohydrin according to the invention may also be obtained when glycerol is heated under a pressure greater than or equal to 1 bar absolute, at a temperature greater than or equal to 150°C, in the presence of a reducing agent, such as a metal sulphite or phosphite for example.

The glycerol may have been obtained starting from fossil raw materials and/or renewable raw materials, preferably starting from renewable raw materials, such as defined in Application WO 2006/100312 in the name of Solvay SA, from page 4, line 18 to page 5, line 24. Glycerol obtained starting from renewable raw materials is, for example, glycerol obtained in processes for conversion of animal or vegetable oils and/or fats, such as hydrolysis, saponification, transesterification, aminolysis or hydrogenation processes and enzymatic rupture processes, such as transesterification or hydrolysis with lipase-type enzymes, such as described in "Medium and Long-Term Opportunities and Risks of the Biotechnological Production of Bulk Chemicals from Renewable Resources, The Potential of White Biotechnology, The BREW Project, Final report Prepared under the European Commission's GROWTH Programme (DG Research), Utrecht, September 2006, pp. 29-31". Glycerol obtained starting from renewable raw materials is, for example, glycerol obtained in processes for conversion of mono- and polysaccharides and derived alcohols, such as fermentation, and thermochemical process such as hydrogenation and hydrogenolysis, as described in "Industrial Bioproducts: Today and Tomorrow, Energetics, Incorporated for the U.S. Department of Energy, Office of Energy Efficiency and Renewable Energy, Office of the Biomass Program, July 2003, pages 49, 52 to 56". The mono- and polysaccharides, such as for example, starch, cellulose and hemicellulose may themselves be obtained from biomass.

In the process of the invention the glycerol-based product can be subjected to at least one treatment, optionally under reduced pressure, chosen from evaporative concentration, evaporative crystallization, distillation, fractional distillation, stripping or liquid/liquid extraction operations.

The term "evaporative concentration" is intended to denote a process of partial evaporation of the product which makes it possible to concentrate the residual product to less volatile entities. The term "evaporative crystallization" is intended to denote a process resulting in the crystallization of a compound by removing, by evaporation, a compound which promotes its dissolution in the medium. These processes are described in "Perry's Chemical Engineers' Handbook" in the 11th section of the 7th edition.

The term "distillation" is intended to denote the type of separation conventional in chemical engineering and described, for example, in "Perry's Chemical Engineers' Handbook" in the 13th section of the 7th edition.

The term "fractional distillation" is understood to mean a series of distillations where the distillate is withdrawn batchwise.

The term "stripping" is intended to denote the separation of a substance by entrainment using the vapour of a pure material. In the process according to the invention, this material can be any compound which is inert with respect to glycerol, such as, for example, steam, air, nitrogen and carbon dioxide.

The term "liquid/liquid extraction" is understood to mean bringing into contact with an appropriate completely or partially immiscible solvent which makes it possible to selectively extract the desired compounds, optionally according to a countercurrent process, such as described in "Perry's Chemical Engineers' Handbook" in the 15th section of the 7th edition.

The stripping, evaporative concentration, evaporative crystallization, liquid/liquid extraction and distillation treatments may be combined, for example in a stripping column surmounted by a distillation section or in a partial evaporator supplying a distillation column or by combining a liquid/liquid extraction, stripping of the residual solvent contained in the glycerol-enriched stream and distillation of the solvent enriched with extracted compounds.

The diols, monoalcohols and the glycerol alkyl ethers are recovered in the distilled, evaporated or stripped fraction and the purified glycerol-based product constitutes an intermediate cut from the distillation, evaporation or stripping treatment.

When the treatment consists of a partial evaporation of the product, the temperature of the glycerol-rich zone is generally greater than or equal to 0°C, often greater than or equal to 80°C and frequently greater than or equal to 100°C. This temperature is generally less than or equal to 280°C, often less than or equal to 250°C, and frequently less than or equal to 200°C. The temperature in the glycerol-depleted zones is generally greater than or equal to -20°C, preferably greater than or equal to -10°C, particularly preferably greater than or equal to 0°C. This temperature is generally at most equal to the temperature of the glycerol-rich zone, preferably at least 5°C below this temperature, particularly preferably at least 10°C below this temperature.

When the treatment is carried out by liquid/liquid extraction, the temperature is generally greater than or equal to 20°C, preferably greater than or equal to 40°C, more particularly greater than or equal to 50°C. This temperature is generally less than or equal to 200°C, preferably less than or equal to 150°C and more particularly preferably less than or equal to 120°C.

The treatment pressure is generally greater than or equal to 0.001 mbar. This pressure is generally less than or equal to 1 bar, often less than or equal to 0.5 bar, frequently less than or equal to 0.3 bar and more specifically less than or equal to 0.25 bar. When the treatment comprises a separate evaporation step, the latter is generally carried out at a pressure less than or equal to 2 bar absolute, preferably at a pressure less than or equal to 1 bar absolute, particularly preferably at a pressure less than or equal to 0.5 bar absolute. It is generally carried out at a pressure greater than or equal to 0.1 mbar, preferably at a pressure greater than or equal to 0.2 mbar. When the evaporation step is combined with a distillation or fractional distillation step, it is carried out at a pressure at least equal to the pressure of the step carried out at the lowest pressure, preferably at a pressure at least 10 mbar greater than the pressure of the step carried out at the lowest pressure. The stripping step is generally carried out at a pressure less than or equal to 5 bar, preferably less than or equal to 2 bar.

In the distillation treatments with or without stripping, the reboiler ratio is generally greater than or equal to 1%, often greater than or equal to 5% and frequently greater than or equal to 10%. This reboiler ratio is less than or equal to 99% and often less than or equal to 50%. The expression " reboiler ratio" is understood to mean, for a continuous distillation, the flow of the vaporized fraction to the reboiler over the flow of the residue.

The expression "reboiler ratio" is understood to mean, for a batchwise, fractional distillation, the ratio of the amount vaporized relative to the final residue.

The amount of the fraction distilled is generally less than or equal to 300 g/kg, often less than or equal to 100g/kg of the glycerol-based product.

The distillation, fractional distillation or stripping treatment may be preceded or followed by an operation which may, for example, be a settling, centrifugation, filtration, adsorption or ion-exchange operation. When it is a settling operation, the operation can be improved by passing through a coalescer. The adsorption operation is preferably an operation for adsorption on activated carbon.

After the treatment, a purified glycerol-based product that comprises at least 1,3-propanediol is obtained, in an amount generally less than or equal to 1 g/kg of purified product. This amount is usually less than 0.7 g/kg, commonly less than or equal to 0.5 g/kg, in many cases less than or equal to 0.1 g/kg of purified product, specifically less than or equal to 0.01 g/kg and particularly less than or equal to 0.001 g/kg. This content is generally greater than or equal to 0.01 mg/kg.

The purified glycerol-based product can also contain, in addition, at least one compound selected from glycerol alkyl ethers, monoalcohols, water, ketones, aldehydes, alkyl esters of fatty acids, glycerol esters, carboxylic acids, glycerol oligomers, salts, compounds containing nitrogen, metallic elements, non-metallic elements, and any mixture of at least two of them. The nature of those compounds and their content in the purified glycerol-based product are as described above for the glycerol-based product.

The invention also relates to a process for manufacturing epichlorohydrin from dichloropropanol, starting from glycerol, in which a glycerol-based product that comprises at least 1,3-propanediol is subjected to at least one treatment, optionally under reduced pressure, chosen from evaporative concentration, evaporative crystallization, distillation, fractional distillation, stripping or liquid/liquid extraction operations, so as to reduce the 1,3-propanediol content and to obtain a purified product which is reacted with a chlorinating agent.

The chlorinating agent generally comprises hydrogen chloride. The hydrogen chloride can be gaseous hydrogen chloride, an aqueous hydrogen chloride solution or a mixture of the two.

The chlorinating agent is such as described in Patent Applications WO 2007/144335, from page 12, line 18 to page 13, line 35, WO 2005/054167, from page 4, line 32 to page 5, line 19, and WO 2006/106153, from page 2, line 10 to page 3, line 20, in the name of Solvay SA.

The reaction with the chlorinating agent may be carried out in the presence of a catalyst, preferably a carboxylic acid or a carboxylic acid derivative, such as described in Patent Application WO 2005/054167, from page 6, line 24 to page 7, line 35 in the name of Solvay SA, and in Application WO 2006/020234, from page 8, line 24 to page 9, line 10, and from page 13, line 1 to page 18, line 3.

The chlorination reaction is preferably carried out in a liquid reaction medium.

The chlorination reaction may be carried out in the presence of a solvent.

The dichloropropanol formed can be separated from the other constituents of the reaction medium by any separation treatment, for example by distillation, stripping, extraction or adsorption. After this treatment, the other constituents of the reaction medium can be subjected to additional separation treatments, such as, for example, a filtration where fatty acid salts can be separated.

When the separation treatment is a distillation, the dichloropropanol separated can be contaminated by 3-chloro-1-propanol, 1,3-dichloropropane, and any mixture thereof. These compounds -may have boiling points close to the dichloropropanol and/or form azeotropes with water that have a boiling point close to that of the dichloropropanol/water azeotrope, making it difficult to separate them from the dichloropropanol. The treatment of the glycerol-based product used in the process for manufacturing epichlorohydrin according to the invention exhibits the advantage of reducing the contamination of the dichloropropanol by these compounds. In the process for manufacturing epichlorohydrin, another part of the dichloropropanol may be obtained by a process other than glycerol chlorination. This process may be chosen from allyl chloride hypochlorination, allyl alcohol chlorination processes, and any combination thereof.

When the dichloropropanol is contaminated by various isomers of chloropropanol, and/or dichloropropane, the epichlorohydrin may be contaminated by dichloropropanes, usually by dichloropropanes and commonly by 1,3-dichloropropane. This 1,3-dichloropropane, has boiling points very close to that of epichlorohydrin and it is therefore very difficult to separate it from epichlorohydrin. This contamination can be particularly troublesome, for example, when epichlorohydrin is used in applications requiring the absence of hydrolysable chloro compounds such as the manufacture of epoxy resins for the electronics industry. This contamination can be reduced by using a dichloropropanol manufactured from purified glycerol-based product described above.

The invention also relates to a process for manufacturing epoxy derivatives such as epoxy resins, glycidyl ethers, glycidyl esters, glycidyl amides and imides, of products which will be used in food and drink applications such as coagulants and wet-strength resins, of cationization agents, of flame retardants, of products which will be used as detergent ingredients, and of epichlorohydrin elastomers, in which epichlorohydrin, at least one part of which was obtained in the process for manufacturing epichlorohydrin according to the invention, is used. The epichlorohydrin is usually subjected to a reaction with at least one compound containing at least one active hydrogen atoms. These compounds comprise polyphenols, monoamines and diamines, aminophenols, heterocyclic imides and amides, diols including ethylene glycol and propylene glycol and aliphatic polyols, and fatty acid dimers. In the process for manufacturing epoxy derivatives such as epoxy resins, glycidyl ethers, glycidyl esters, glycidyl amides and imides, of products which will be used in food and drink applications such as coagulants and wet-strength resins, of cationization agents, of flame retardants, of products which will be used as detergent ingredients, and of epichlorohydrin elastomers, another part of the epichlorohydrin may be obtained by a process other than the dehydrochlorination of dichloropropanol derived from the chlorination of glycerol, such as a process for dehydrochlorination of dichloropropanol derived from hypo chlorination of allyl chloride, a process for dehydrochlorination of dichloropropanol derived from chlorination of allyl alcohol, or an allyl chloride epoxidation process.

The epoxy resins thus obtained may be used in coating applications and in structural applications. The coating applications relate to the fields of maritime transport and of industrial maintenance (anticorrosion primer paints for metal and concrete structures), of coatings for metallic containers (food preserves, cans for drinks, drums, buckets and aerosol bottles) and for windings, of motor vehicle coatings (primers), of inks and masks for electronic circuits. The structural applications relate to the fields of structural composites (epoxy resin composites with glass, boron, carbon and aromatic polyamide fibres), of civil engineering, of floor covering (paints for coating floors, parquet, paving, tiling, self-levelling coatings, roughcast floors, tempered floors, floor coverings for cold rooms), of construction, of electrical equipment (sealing of electrical and electromechanical devices such as battery housings, resistors, fuses, thermal circuit breakers, cable joints, windings) and of electronic equipment (coatings and laminated sheets for printed circuits and encapsulation of printed circuits), of adhesives (bonding of different materials such as metals, glass, ceramics, wood, concrete, plastics) and of tooling (prototypes, master patterns, moulds and other parts) for the aerospace, automotive, foundry and maritime construction industries.

Epoxy resins also find applications in the fields of energy (wind energy), of aeronautics (honeycomb sandwich panels, helicopter rotor blades, cowls and engine nacelles, flaps, ailerons, rudders) and of fluid (gas, oil) transport.

Glycidyl esters and ethers are generally used for applications such as coatings, adhesives and reactive diluents.

Glycidyl amines and imides are usually used for applications such as outdoor powder coatings with polyesters, or in applications in which a non-yellowing epoxy resin is desirable.

Coagulants can be used for treatment of raw water for conversion to drinking water, for recycling paper of water in Pulp & Paper Industry, for paint detackification, for breaking oil emulsions, for oil and grease removal, and for sludge dewatering. They can also be used for sugar refining.

Wet-strength resins can be used in papers that will get wet such as paper towels, tea bags, coffee filters, milk cartons, meat wrapping, and wallpaper. They can also be used in the production of high fructose com syrup and to prevent wool from shrinking.

Cationization agents are mainly used in the cationization of starch to be utilized by the paper industry for processing of high quality paper grades or for cationization of textile for dye fixing.

Flame retardants are usually used to inhibit the evolution of combustible gases in various materials such as polymers, in particular in polyurethane foams.

Examples of detergent ingredients are surfactants or surface deposition enhancing materials. They are usually used as components of cleaning compositions for instance dishwashing, laundry compositions, shampoos and synbars.

The epichlorohydrin elastomers are generally used in specialty applications, like for instance automotive components (fuel pump diaphragms, emission control hoses, motor mounts, gaskets, seals and portable fuel tanks), in the aircraft industry, for specialty roofing membranes, coated fabrics, solvent storage containers, paper mill and printing roll and in a variety of oil specialties.

The examples below are intended to illustrate the invention without, however, limiting it.

### Example 1

A reactor has been fed continuously with glycerol containing 1.5 g of 1,3-propanediol per kg and 30 g of adipic acid per kg, at a rate of 14.3 g/h/l of reactor, with gaseous hydrogen chloride at rate of 11.3 g/h/l of reactor. The reactor has been operated at a temperature of 117 °C and at a pressure of 1.14 bar. The reactor was surmounted by a distillation column from which dichloropropanol containing water has been withdrawn. The liquid reaction mixture has been withdrawn from the reactor and has been sent to a distillation unit to recover dichlororopanol.

The dichloropropanol which has been recovered from both withdrawals contained 1.67 g of 3-chloro-1-propanol and 0.44 g of 1,3-dichloropropane per kg of dichloropropanol.

### Example 2

The procedure of example 1 has been followed except that the glycerol contained 0.005 g of 1,3-propanediol per kg.

A reactor has been fed continuously with glycerol containing 0.005 g of 1,3-propanediol per kg and 12 g of adipic acid per kg, at a rate of 21.5 g/h/l of reactor, with gaseous hydrogen chloride at rate of 17 g/h/l of reactor. The reactor has been operated at a temperature of 119 °C and at a pressure of 1.19 bar. The reactor was surmounted by a distillation column from which dichloropropanol containing water has been withdrawn. The liquid reaction mixture has been withdrawn from the reactor and has been sent to a distillation unit to recover dichlororopanol.

The dichloropropanol which has been recovered from both withdrawals contained 0.011 g of 3-chloro-1-propanol and less than 0.010 g of 1,3-dichloropropane per kg of dichloropropanol.

### Example 3

A well stirred reactor has been fed continuously with dichloropropanol containing 0.223 g of 1,3-dichloropropane and 2.93 g of 3-chloro-1-propanol per kg of dichloropropanol at a rate of 5.07 kg/h/l of reactor, with a sodium hydroxide solution containing 500 g of NaOH per kg at rate of 2.83 kg/h/l of reactor and with recycled water at a rate of 6.23 kg/h/l of reactor. The reactor has been operated at a temperature of 45 °C and at a pressure of 1 bar. The reactor was fitted with an overflow system for withdrawal of the liquid reaction medium. The liquid reaction medium was biphasic. The lighter phase which contained the salt and a small quantity of organics has been stripped with steam and the condensate has been recycled to the reactor. The heavier phase which contained the major part of the epichlorohydrin formed in the reactor has been submitted continuously to a first distillation operation under vacuum in a packed column to separate epichlorohydrin, water and the low boiling point byproducts at the top of the column and the excess of dichloropropanol and the heavy by-products at the bottom of the column. The top fraction, collected at a temperature of 60.8°C under a pressure of 150 mbar, has been submitted continuously to a second distillation in a packed column to eliminate water and the low boiling by-products. The bottom product has been collected at normal pressure at a temperature of 115.2°C; it contained 0.35 g of 1,3-dichloropropane per kg. This product has been purified by a third operation of distillation that has been realized in a batchwise manner in a glass plate distillation column under vacuum at a pressure of 790 mbar. The epichlorohydrin which has been recovered as the main distillation fraction collected between 107.4 - 107.6°C contained 0.34 g of 1,3-dichloropropane per kg and is named ECH 2.

### Example 4

A well stirred reactor has been fed continuously with dichloropropanol containing 0.013 g of 1,3-dichloropropane and 0.0026 g of 3-chloro-1-propanol per kg of dichloropropanol at a rate of 5.93 kg/h/l of reactor, with a sodium hydroxide solution containing 500 g of NaOH per kg at rate of 2.67 kg/h/l of reactor and with recycled water at a rate of 5.83 kg/h/l of reactor. The reactor has been operated at a temperature of 45 °C and at a pressure of 1 bar. The reactor was fitted with an overflow system for withdrawal of the liquid reaction medium. The liquid reaction medium was biphasic. The lighter phase which contained the salt and a small quantity of organics has been stripped with steam and the condensate has been recycled to the reactor. The heavier phase which contained the major part of the epichlorohydrin formed in the reactor has been submitted continuously to a first distillation operation in a packed column to separate epichlorohydrin, water and the low boiling point byproducts at the top of the column and the excess of dichloropropanol and the heavy by-products at the bottom of the column. The top fraction, collected at a temperature of 62.4°C under a pressure of 150 mbar, has been submitted continuously to a second distillation in a packed column to eliminate water and the low boiling by-products as the top product and some heavy impurities at the bottom of the column. The epichlorohydrin has been collected by condensation of a gas phase collected from the middle of the column at normal pressure and at a temperature of 116.4 °C ; it contained 0.03 g of 1,3-dichloropropane per kg and is named ECH 5.

The complete composition of epichlorohydrins ECH 2 and ECH 5 and of three other epichlorohydrin samples (ECH 1, ECH 3 and ECH 4) are presented in Table 1.

Their compositions obtained by gas chromatography analysis.

**Table 1**

| **Component (g/kg)** | **ECH1** | **ECH 2** | **ECH3** | **ECH4** | **ECH5** |
|---|---|---|---|---|---|
| Acetaldehyde | 0.004 | n.d | n.d | n.d. | n.d. |
| Acrolein | < 0.001 | 0.003 | 0.003 | n.d. | n.d. |
| 2-propanol | < 0.001 | n.d. | n.d. | n.d. | n.d. |
| 3-chloro-1-propene | n.d. | n.d. | n.d. | n.d. | n.d. |
| allyl alcohol | 0.001 | < 0.001 | < 0.001 | n.d. | 0.003 |
| Hydroxyacetone | 0.094 | 0.018 | 0.018 | 0.006 | 0.006 |
| chloroacetone + (3,3-dichloro-1-propene) | 0.033 | 0.038 | 0.040 | n.d. | 0.024 |
| 1,2-dichloropropane | 0.042 | n.d. | n.d. | 0.001 | n.d. |
| 2,3-dichloro-1-propene | 0.005 | n.d. | n.d. | 0.004 | n.d. |
| 1-chloro-2, 3-epoxypropane (*) | > 998.464 | > 999.474 | > 999.045 | > 999.503 | > 999.865 |
| 1,3-dichloro-1-propene cis maj. + (C6Hl4O min.) | 0.219 | 0.008 | 0.008 | 0.032 | 0.004 |
| 2-chloro-2-propene-1-ol | 0.348 | 0.016 | 0.016 | 0.14 | 0.012 |
| 1,3-dichloro-1-propene trans | 0.035 | 0.010 | 0.010 | 0.008 | 0.009 |
| C₅H₁₀O / C₄H₇ClO | n.d. | n.d. | n.d. | 0.014 | 0.001 |
| C₆H₁₂O | n.d. | n.d. | n.d. | 0.011 | < 0.001 |
| 1,3-dichloropropane | 0.002 | 0.34 | 0.34 | 0.005 | 0.030 |
| Cyclopentanone | 0.001 | 0.004 | 0.004 | n.d. | 0.004 |
| dibromochloromethane | 0.004 | n.d. | n.d. | 0.084 | n.d. |
| C₆H₁₀O iso 1 | 0.003 | n.d. | n.d. | 0.009 | < 0.001 |
| C₆H₁₀O iso 2 | 0.012 | n.d. | n.d. | 0.009 | 0.001 |
| 1,2-epoxyhexane + (1,2,2-trichloropropane) | 0.030 | 0.002 | 0.002 | n.d. | 0.001 |
| C₆H₁₀O iso 3 | 0.004 | n.d. | n.d. | 0.031 | 0.001 |
| dichloroepoxypropane + Ni | 0.003 | n.d. | n.d. | 0.006 | n.d. |
| 1,3,3-trichloro-1-propene cis + 1,1,3-trichloropropene | 0.012 | n.d. | n.d. | 0.004 | n.d. |
| 1,1,2-trichloropropane | 0.211 | 0.001 | 0.001 | 0.025 | 0.007 |
| chlorobenzene | 0.011 | < 0.001 | < 0.001 | 0.001 | 0.007 |
| 1,3,3-trichloro-1-propene trans | 0.015 | n.d. | n.d. | 0.012 | 0.001 |
| 1,2,3-trichloropropene trans | 0.016 | < 0.001 | < 0.001 | 0.003 | 0.001 |
| 1,3-dichloro-2-propanol | 0.111 | 0.023 | 0.024 | 0.017 | 0.008 |
| 1,2,3-trichloropropane | 0.014 | n.d. | n.d. | 0.024 | n.d. |
| 1,2,3-trichloropropene cis | 0.002 | n.d. | n.d. | n.d. | n.d. |
| 3-chloro-1,2-propanediol + 2,3-dichloro-1-propanol | 0.13 | < 0.001 | 0.001 | n.d. | 0.001 |
| C₆H₁₃Br | n.d. | n.d. | n.d. | 0.005 | n.d. |
| C₆H₁₀Cl₂ iso 1 | n.d. | n.d. | n.d. | 0.005 | n.d. |
| C₆H₁₀Cl₂ iso 2 | n.d. | n.d. | n.d. | 0.004 | n.d. |
| methyl glycidyl ether | 0.007 | 0.054 | 0.48 | n.m. | n.m. |
| Unknowns (sum) | 0.170 | 0.007 | 0.008 | 0.087 | 0.024 |

| | | | | | |
|---|---|---|---|---|---|
| n.d. : not detected, n.m. : not measured *: 1-chloro-2, 3-epoxypropane amount calculated on the basis of the total content of other organic components | | | | | |

### Examples 5 to 14 (homopolymerization of ECH)

The tests have been carried out according to the following procedure with epichlorohydrin sample ECH1 (examples 5 to 7), ECH 2 (examples 8 to 10) and ECH 3 (examples 11 to 14). The quantities of chemicals are indicated in Table 2.

The polymerization of epichlorohydrin (ECH) has been carried out in the presence of the system tetraoctylammonium bromide (Noct₄Br)/triisobutyl aluminium (TiBA).

The epichlorohydrin has been dried over calcium hydride under vacuum for 24 h at 25 °C and further distilled.

The polymerization reactions have been carried out in pyrex vessels fitted with polytetrafluorethylene valves. The vessels have been evacuated under flame heating to remove residual moisture. After cooling to room temperature, the vessels have been cooled to - 30 °C (ethanol/liquid nitrogen cooling bath) and toluene and epichlorohydrin, have been added under vacuum. After those additions, argon has been introduced in the vessel and tetraoctylammonium bromide and triisobutyl aluminium have been added to the vessel. This addition constituted the time zero of the reaction. After a given time under magnetic stirring at - 30 °C, the reaction has been stopped by adding 1-2 ml of ethanol to the vessel. Half of the volume of the reaction medium has then been submitted to evaporation after which the polymer has been recovered from the vessel.

The conversion has been obtained by comparing the weight of recovered polymer with the weight of added epichlorohydrin.

The theoretical molar weight (Mn th.) has been calculated on the basis of the quantity of tetraoctylammonium bromide.

The measured polymer molar weight (Mn exp) and the molar weight dispersion have been obtained by Gel Permeation Chromatography.

The tacticity of the polymer has been obtained by ¹³C and ¹H NMR.

The results of the tests are summarized in Table 3.

**Table 2**

| **Example n°** | **ECH (ml)** | **Toluene (ml)** | **Noct₄Br (ml)** | **TiBA (ml)** |
|---|---|---|---|---|
| 5 | 4 | 10.2 | 2.15 | 0.71 |
| 6 | 4 | 10.2 | 2.15 | 0.71 |
| 7 | 3.4 | 9.9 | 0.91 | 0.30 |
| 8 | 4 | 10.2 | 2.15 | 0.71 |
| 9 | 4 | 10.2 | 2.15 | 0.71 |
| 10 | 4 | 11.6 | 1.08 | 0.35 |
| 11 | 4 | 10.2 | 2.15 | 0.71 |
| 12 | 4 | 10.2 | 2.15 | 0.71 |
| 13 | 3.6 | 11.4 | 0.97 | 0.43 |
| 14 | 4 | 11.6 | 1.08 | 0.35 |

**Table 3**

| **Example** | **Reaction time (h)** | **Conversion (mol %)** | **Mn th**. **(g/mol)** | **Mn exp.** (**g**/**mol**) | **Dispersion** | **Tacticity** |
|---|---|---|---|---|---|---|
| 5 | 1 | 100 | 10000 | 10700 | 1.17 | atactic |
| 6 | 1 | 100 | 10000 | 10100 | 1.23 | n.m. |
| 7 | 2 | 100 | 20000 | 20200 | 1.17 | n.m. |
| 8 | 1 | 100 | 10000 | 16400 | 1.22 | n.m. |
| 9 | 1 | 100 | 10000 | 11200 | 1.20 | atactic |
| 10 | 1 | 100 | 20000 | 77700 (20%) | | n.m. |
| | | | | 22200 (80%) | | |
| 11 | 1 | 80 | 8000 | 6800 | 1.17 | n.m. |
| 12 | 2 | 95 | 9500 | 12100 | 1.17 | atactic |
| 13 | 2 | 90 | 18000 | 24700 | 1.18 | n.m. |
| 14 | 6 | 94 | 18800 | 17650 | 1.17 | n.m. |

| | | | | | | |
|---|---|---|---|---|---|---|
| n.m. : not measured | | | | | | |

### Examples 15 to 17 (homopolymerization of ECH)

The tests have been carried out according to the following procedure with epichlorohydrin sample ECH1 (example 15), ECH 2 (example 16) and ECH 3 (example 17). The quantities of chemicals are indicated in Table 4.

The polymerization of epichlorohydrin (ECH) has been carried out in the presence of the system water/triethyl aluminium (TEA).

The procedure of example 1 has been followed except that TEA in solution in toluene and water have been added under argon to the vessel first evacuated and dried, left under magnetic stirring under vacuum for 30 min, before ECH in toluene has been added (time zero of the reaction). The polymerization has been carried out at a temperature of 25 °C for 12 h. The results have been summarized in Table 5

**Table 4**

| **Example n°** | **ECH (ml)** | **Toluene (ml)** | **H20 (µl)** | **TEA (ml)** |
|---|---|---|---|---|
| 15 | 4 | 10 | 23 | 0.67 |
| 16 | 4 | 10 | 23 | 0.67 |
| 17 | 4 | 10 | 23 | 0.67 |

**Table 5**

| **Example** | **Reaction time (h)** | **Conversion (mol %)** | **Mn exp. (g/mol)** | **Dispersion** | **Tacticity** |
|---|---|---|---|---|---|
| 15 | 12 | 47 | 216000 | 2.02 | n.m. |
| | | | 7000 | 1.04 | |
| 16 | 12 | 50 | 285200 | 3.51 | atactic |
| | | | 5850 | 1.08 | |
| 17 | 12 | 55 | 357600 | 3.45 | atactic |
| | | | 8100 | 1.31 | |

| | | | | | |
|---|---|---|---|---|---|
| n.m. : not measured | | | | | |

### Example 18

### Preparation of a product consisting predominantly in diglycidyl diether of Bisphenol A according to US 2,811,227

The apparatus employed was a thermostatised flask equipped with a mechanical stirrer, with a jacket containing a thermocouple and with a Dean-Stark separator surmounted by a water-cooled condenser. A pump was used to inject a caustic soda aqueous solution at a constant rate in the flask.

The reaction flask was initially charged with a mixture of bisphenol A (68.4 g, 0.3 mol) and the epichlorohydrin sample ECH4 coming from a propylene-chlorine plant (277.5 g, 3.0 mol). The analysis of the epichlorydrin is given in Table 1. The mixture was heated at reflux under stirring to a temperature of 111°C. A 40 % aqueous solution of caustic soda (60.8 g, 0.6 mol) was introduced at a rate of 12 ml/h during 3.5 hour. The temperature of the mixture in the flask was maintained in the range 100 °C - 115 °C in order to assure a constant reflux. The epichlorohydrin rich organic phase decanted during the reaction as a lower phase in the separator was recycled regularly in the reaction flask and the aqueous rich phase collected as an upper phase in the separator was regularly drawn off. The heating was maintained for 15 min after the total introduction of the caustic soda solution to achieve the collect of the water phase in the decantor. 29.7 g of aqueous phase was collected with a composition given in Table 6.

The epichlorohydrin in excess was removed from the reaction mixture by distillation under a vacuum of 30 mbar and by a progressive heating of the mixture to 109°C. 156.1 g (1.7 mol) of epichlorohydrin was recovered in this step. The composition of the distillate is given in Table 6.

The salt was separated from the crude product (45.5 g) after addition of 567.2 g of toluene under agitation and by filtration. The cake of filtration was washed with 124.4 g of toluene. The toluene solutions were mixed and evaporated at 185°C under a pressure of 1 mbar.

659.4 g of toluene was recovered as the condensate of the evaporated fraction with a composition given in Table 6. The residual product of the evaporation (100.5 g) contained the diglycidyl ether of bis-phenol A as a major product and no trace of unconverted bis-phenol A (< 5 mg/kg). The residue contained 4.98 mol epoxy per kg and 1.52 % of hydrolysable chlorine.

### Example 19

The trial was realized in the apparatus described in example 18.

The reaction flask was initially charged with a mixture of bisphenol A (68.4 g, 0.3 mol) and epichlorohydrin sample ECH 5 (277.5 g, 3.0 mol). The analysis of the epichlorydrin is given in Table 1. The mixture was heated at reflux under stirring to a temperature of 119°C. A 40 % aqueous solution of caustic soda (60.8 g, 0.6 mol) was introduced at a rate of 12 ml/h during 3.5 hour. The temperature of the mixture in the flask was maintained in the range 102 °C - 119 °C in order to assure a constant reflux. The epichlorohydrin rich organic phase decanted during the reaction as a lower phase in the separator was recycled regularly in the reaction flask and the aqueous rich phase collected as an upper phase in the separator was regularly drawn off. The heating was maintained for 15 min after the total introduction of the caustic soda solution to achieve the collect of the water phase in the decantor. 54.5 g of aqueous phase was collected with a composition given in Table 6.

The epichlorohydrin in excess was removed from the reaction mixture by distillation under a vacuum of 30 mbar and by a progressive heating of the mixture to 118°C. 148.2 g (1.5 mol) of epichlorohydrin was recovered in this step. The composition of the distillate is given in Table 6.

The salt was separated from the crude product (47.8 g) after addition of 228.4 g of toluene under agitation and by filtration. The cake of filtration was washed with 97.3 g of toluene. The toluene solutions were mixed and evaporated at 180°C under a pressure of 1 mbar.

305.0 g of toluene was recovered as the condensate of the evaporation with a composition given in Table 6. The residual product of the evaporation (99.8 g) contained the diglycidyl ether of bis-phenol A as a major product and no trace of unconverted bis-phenol A (< 5 mg/kg). The residue contained 4.93 mol epoxy per kg and 0.49 % of hydrolysable chlorine.

The High Performance Liquid Chromatography analyses of the residual products obtained in examples 18 and 19 are similar.

**Table 6**

| **Component** | **Example 18** | | | **Example 19** | | |
|---|---|---|---|---|---|---|
| | Epichlorohydrin evaporated (g/kg) | Water evaporated (mg/l) | Toluene evaporated (g/kg) | Epichlorohydrin evaporated (g/kg) | Water evaporated (mg/l) | Toluene evaporated (g/kg) |
| acetaldehyde | n.d. | 2.9 | n.d. | n.d. | 1.3 | n.d. |
| acrolein | n.d. | 0.58 | n.d. | 0.002 | 0.42 | n.d. |
| 2-propanol | n.d. | < 0.05 | n.d. | n.d. | 0.3 | n.d. |
| 3-chloro-1-propene | 0.001 | n.d. | n.d. | n.d. | n.d. | n.d. |
| allyl alcohol | n.d. | n.d. | n.d. | 0.001 | 0.2 | n.d. |
| hydroxyacetone | 0.016 | n.d. | n.d. | 0.002 | n.d. | n.d. |
| chloroacetone + (3,3-dichloro-1-propene) | 0.003 | 0.65 | n.d. | 0.002 | 0.53 | n.d. |
| 1,2-dichloropropane | | | n.d. | n.d. | n.d. | n.d. |
| 2,3 -dichloro-1-propene | 0.005 | 0.07 | n.d. | n.d. | n.d. | n.d. |
| 1-chloro-2,3-epoxypropane | principal product | (45g/kg) | 1.6 | principal product | (46g/kg) | 3.3 |
| 1,3-dichloro-1-propene cis maj. + (C6Hl4O min.) | 0.026 | 0.36 | n.d. | 0.003 | n.d. | n.d. |
| 2-chloro-2-propene-1-ol | 0.19 | 0.12 | n.d. | 0.016 | < 0.05 | n.d. |
| 1,3-dichloro-1-propene trans | 0.007 | < 0.05 | n.d. | 0.008 | n.d. | n.d. |
| C₅H₁₀O / C₄H₇ClO | 0.019 | 0.05 | n.d. | 0.001 | n.d. | n.d. |
| C₆H₁₂O | 0.022 | 0.28 | n.d. | 0.021 | n.d. | n.d. |
| 1,3-dichloropropane | 0.001 | n.d. | n.d. | 0.03 | < 0.05 | n.d. |
| Cyclopentanone | n.d. | n.d. | n.d. | 0.006 | n.d. | n.d. |
| dibromochloromethane | 0.080 | n.d. | n.d. | n.d. | n.d. | n.d. |
| C₆H₁₀O iso 1 | 0.033 | 0.11 | n.d. | 0.038 | n.d. | n.d. |
| C₆H₁₀O iso 2 | 0.040 | 0.31 | n.d. | 0.001 | n.d. | n.d. |
| 1,2-epoxyhexane + (1,2,2-trichloropropane) | n.d. | n.d. | n.d. | 0.001 | n.d. | n.d. |
| C₆H₁₀O iso 3 | 0.036 | 0.21 | n.d. | 0.002 | n.d. | n.d. |
| dichloroepoxypropane | 0.006 | | n.d. | n.d. | n.d. | n.d. |
| 1,3,3-trichloro-1-propene cis + 1,1,3-trichloropropene | 0.006 | n.d. | n.d. | n.d. | n.d. | n.d. |
| 1,1,2-trichloropropane | 0.005 | n.d. | n.d. | | n.d. | n.d. |
| chlorobenzene | 0.001 | n.d. | n.d. | 0.008 | n.d. | n.d. |
| 1,3,3-trichloro-1-propene trans | 0.009 | n.d. | n.d. | n.d. | n.d. | n.d. |
| 1,2,3-trichloropropene trans | 0.003 | n.d. | n.d. | 0.001 | n.d. | n.d. |
| 1,3 -dichloro-2-propanol | 3.4 | 143 | 0.38 | 2.5 | 111 | 0.074 |
| 1,2,3-trichloropropane | 0.022 | n.d. | 0.002 | n.d. | n.d. | n.d. |
| 1,2,3-trichloropropene cis | n.d. | n.d. | n.d. | n.d. | n.d. | n.d. |
| 3-chloro-1,2-propanediol + 2,3-dichloro-1-propanol | 0.13 | 5.9 | 0.064 | 0.071 | 4.1 | 0.033 |
| C₆H₁₃Br | n.d. | < 0.05 | 0.005 | n.d. | < 0.05 | |
| C₆H₁₀Cl₂ iso 1 | 0.009 | n.d. | n.d. | n.d. | n.d. | n.d. |
| C₆H₁₀Cl₂ iso 2 | 0.007 | n.d. | n.d. | n.d. | n.d. | n.d. |
| methyl glycidyl ether | n.m. | n.m. | n.m. | n.m. | n.m. | n.m. |
| Unknowns (sum) | 0.299 | 10.00 | 1.31 | 0.213 | 1.3 | 1.373 |

| | | | | | | |
|---|---|---|---|---|---|---|
| n.d. : not detected, n.m. : not measured | | | | | | |

## Claims

1. Process for manufacturing epichlorohydrin, by subjecting dichloropropanol to a dehydrochlorination reaction, in which, at least one part of the dichloropropanol was obtained by reacting a glycerol-based product with a chlorinating agent,
wherein the glycerol-based product contains at least 900 g/kg of glycerol and from 0.001 g/kg to 5 g/kg of 1,3-propanediol.

2. Process according to claim 1 wherein glycerol-based product contains at least 995 g/kg of glycerol.

3. Process according to claim 1 or 2 wherein the glycerol-based product contains from to 0.001 g/kg to 0.7 g/kg of 1,3-propanediol.

4. Process according to any one of Claims 1 to 3, wherein the glycerol-based product comprises, in addition, 1,2-ethanediol and/or 1,2-propanediol.

5. Process according to any one of Claims 1 to 4, wherein the glycerol-based product is subjected to at least one treatment, optionally under reduced pressure, chosen from evaporative concentration, evaporative crystallization, distillation, fractional distillation, stripping or liquid/liquid extraction operations, in order to obtain a purified glycerol-based product, prior to the reaction with the chlorinating agent.

6. Process according to Claim 5, wherein the purified glycerol-based product comprises at least 1,3-propanediol, in an amount less than 0.7 g diol/kg of purified product.

7. Process according to any of Claims 1 to 6, wherein the chlorinating agent comprises hydrogen chloride.

8. Process for manufacturing epoxy resins or glycidyl esters or glycidyl ethers or glycidyl amides or glycidyl imides or coagulants or wet-strength resins or cationization agents or flame retardants or detergent ingredients or epichlorohydrin elastomers, in which epichlorohydrin, at least one part of which was obtained according to the process from any one of Claims 1 to 7, is subjected to a reaction with at least one compound containing at least one active hydrogen atom, wherein the compound containing at least one active hydrogen atom comprises polyphenols, monoamines and diamines, aminophenols, heterocyclic imides and amides, diols including ethylene glycol and propylene glycol and aliphatic polyols, and fatty acid dimers.

## Patentansprüche

1. Verfahren zur Herstellung von Epichlorhydrin durch Dehydrochlorierung von Dichlorpropanol, wobei mindestens ein Teil des Dichlorpropanols durch Umsetzung eines auf Glycerin basierenden Produkts mit einem Chlorierungsmittel erhalten wurde,
wobei das auf Glycerin basierende Produkt mindestens 900 g/kg Glycerin und 0,001 g/kg bis 5 g/kg 1,3-Propandiol enthält.

2. Verfahren nach Anspruch 1, bei dem das auf Glycerin basierende Produkt mindestens 995 g/kg Glycerin enthält.

3. Verfahren nach Anspruch 1 oder 2, bei dem das auf Glycerin basierende Produkt 0,001 g/kg bis 0,7 g/kg 1,3-Propandiol enthält.

4. Verfahren nach einem der Ansprüche 1 bis 3, bei dem das auf Glycerin basierende Produkt außerdem 1,2-Ethandiol und/oder 1,2-Propandiol umfasst.

5. Verfahren nach einem der Ansprüche 1 bis 4, bei dem man das auf Glycerin basierende Produkt vor der Umsetzung mit dem Chlorierungsmittel gegebenenfalls unter vermindertem Druck einer aus Eindampfen, Verdampfungskustallisation, Destillation, fraktionierter Destillation, Strippen oder Flüssig/Flüssig-Extraktionsarbeitsgängen ausgewählten Behandlung unterwirft, wobei man ein gereinigtes auf Glycerin basierendes Produkt erhält.

6. Verfahren nach Anspruch 5, bei dem das gereinigte auf Glycerin basierende Produkt zumindest 1,3-Propandiol in einer Menge von weniger als 0,7 g Diol/kg gereinigtes Produkt umfasst.

7. Verfahren nach einem der Ansprüche 1 bis 6, bei dem das Chlorierungsmittel Chlorwasserstoff umfasst.

8. Verfahren zur Herstellung von Epoxidharzen oder Glycidylestern oder Glycidylethern oder Glycidylamiden oder Glycidylimiden oder Koagulantien oder Nassfestharzen oder Kationisierungsmitteln oder Flammschutzmitteln oder Wasch- und Reinigungs mittelinhaltsstoffen oder Epichlorhydrin-Elastomeren, bei dem man Epichlorhydrin, das zumindest teilweise gemäß dem Verfahren nach einem der Ansprüche 1 bis 7 erhalten wurde, einer Umsetzung mit mindestens einer Verbindung mit mindestens einem aktiven Wasserstoffatom unterwirft, wobei die Verbindung mit mindestens einem aktiven Wasserstoffatom Polyphenole, Monoamine und Diamine, Aminophenole, heterocyclische Imide und Amide, Diole einschließlich Ethylenglykol und Propylenglykol und aliphatische Polyole und Fettsäuredimere umfasst.

## Revendications

1. Procédé de fabrication d'épichlorhydrine, par soumission de dichloropropanol à une réaction de déshydrochloration, au moins une partie du dichloropropanol ayant été obtenue en faisant réagir un produit à base de glycérol avec un agent chlorant,
dans lequel le produit à base de glycérol contient au moins 900 g/kg de glycérol et de 0,001 g/kg à 5 g/kg de 1,3-propanediol.

2. Procédé selon la revendication 1 dans lequel le produit à base de glycérol contient au moins 995 g/kg de glycérol.

3. Procédé selon la revendication 1 ou 2 dans lequel le produit à base de glycérol contient de 0,001 g/kg à 0,7 g/kg de 1,3-propanediol.

4. Procédé selon l'une quelconque des revendications 1 à 3, dans lequel le produit à base de glycérol comprend également du 1,2-éthanediol et/ou du 1,2-propanediol.

5. Procédé selon l'une quelconque des revendications 1 à 4, dans lequel le produit à base de glycérol est soumis à au moins un traitement, optionnellement sous pression réduite, choisi parmi une évapoconcentration, une évapocristallisation, une distillation, une distillation fractionnée, des opérations de stripage ou d'extraction liquide/liquide, afin d'obtenir un produit à base de glycérol purifié, avant la réaction avec l'agent chlorant.

6. Procédé selon la revendication 5, dans lequel le produit à base de glycérol purifie comprend au moins du 1,3-propanediol, dans une quantité inférieure à 0,7 g de diol/kg de produit purifié.

7. Procédé selon l'une quelconque des revendications 1 à 6, dans lequel l'agent chlorant comprend du chlorure d'hydrogène.

8. Procédé de fabrication de résines époxy ou d'esters glycidyliques ou d'éthers glycidyliques ou d'amides glycidyliques ou d'imides glycidyliques ou de coagulants ou de résines résistantes à l'état humide ou d'agents de cationisation ou d'ignifugeants ou d'ingrédients détergents ou d'élastomères d'épichlorhydrine, dans lequel de l'épichlorhydrine, dont au moins une partie a été obtenue selon le procédé de l'une quelconque des revendications 1 à 7, est soumise à une réaction avec au moins un composé contenant au moins un atome d'hydrogène actif, le composé contenant au moins un atome d'hydrogène actif englobant les polyphénols, les monoamines et diamines, les aminophénols, les imides et amides hétérocycliques, les diols y compris l'éthylène glycol et le propylène glycol et les polyols aliphatiques, et les dimères d'acides gras.
